# EUROPÄISCHE PATENTANMELDUNG

(11) **EP 2 742 928 A1**
(43) Veröffentlichungstag der Anmeldung: **18.06.2014**
(21) Anmeldenummer: 13192861.6
(22) Anmeldetag: 14.11.2013
(51) Int. Cl.: A61K 8/34, A61K 8/37, A61K 8/55, A61Q 19/00, A61K 8/81, A61K 8/92

(54) **Emulsion mit antibakteriellem Wirkstoff**

(30) Priorität: 17.12.2012 DE 102012223401
(71) Anmelder: Henkel AG&Co. KGAA, 40589 Düsseldorf (DE)
(72) Erfinder: Bähren, Annika, 41363 Jüchen (DE); Stadler, Iris Marina, 47249 Duisburg (DE); Waldmann-Laue, Marianne, 40789 Monheim (DE)

(57) **Zusammenfassung**

Die Erfindung betrifft kosmetische Zusammensetzungen in Form einer Emulsion, enthaltend
a) mindestens einen antibakteriellen Wirkstoff,
und
b) mindestens ein Salz von C₁₂₋₂₀-Alkylphosphat, insbesondere ein Salz von Cetylphosphat,
und
c) mindestens ein C₁₄₋₂₀-Mono- oder Diacylglycerid, bevorzugt mindestens ein C₁₆₋₁₈-Mono- oder Diacylglycerid, besonders bevorzugt ausgewählt aus gehärteten Palmölglyceriden,
und
d) mindestens ein Polymer, welches durch Polymerisation von zumindest 2-Methyl-2[(1-oxo-2-propenyl)amino]-1-propansulfonsäure (AMPS) erhalten wird
und
e) Wasser.

## Beschreibung

Die Erfindung betrifft emulsionsförmige Zusammensetzungen, die in Kosmetika, beispielsweise für die Haut oder die Haare, Verwendung finden.

Viele wasserhaltige Zusammensetzungen, insbesondere in Form von Kosmetika, umfassen Öl-oder Fettkomponenten. Diese Öl- oder Fettkomponenten sind nicht wasserlöslich und werden daher z.B. in wasserhaltigen Kosmetika meist mit Hilfe von Emulgatoren in Form einer Emulsion eingearbeitet. Öl- oder Fettkomponenten werden einerseits zur Pflege des Substrats, insbesondere der Haut oder Haare, eingesetzt. Andererseits lassen sich in Ölen oder Fetten öllösliche Wirkstoffe, wie z.B. UV-Filter, in ein wasserhaltiges Kosmetikum in gelöster Form einarbeiten. Die in der Öl-oder Fettkomponente gelösten Wirkstoffe können in dieser Form gleichmäßiger auf das Substrat aufgebracht werden. Auf diese Weise kann sich die Wirkung des Wirkstoffes am Substrat verbessert entfalten.

Die Hautmikroflora hat einen entscheidenden Einfluss auf unterschiedliche kosmetische Parameter. So spielen pathogene Keime wie Staphylococcus aureus eine entscheidende Rolle bei der Entstehung von Hautunreinheiten. Neueste Studien deuten auch darauf hin, dass eine unausgeglichene Hautmikroflora einen Einfluss auf die Hautalterung ausüben kann, da unerwünschte Keime zu einer gesteigerten Immunabwehr der Haut führen, die ihrerseits zu vermehrten Entzündungsreaktionen führt, in deren Verlauf Hautalterungsmarker stimuliert werden. Es besteht daher nach wie vor der Bedarf nach Konservierungsmittelzusammensetzungen, die einerseits die Besiedlung des Produktes oder der Haut mit unerwünschten Keimen verhindern und andererseits die natürliche Hautflora nicht oder nicht wesentlich beeinträchtigen.
Die Mischung verschiedener antimikrobieller Substanzen zur Steigerung der antimikrobiellen Aktivität ist grundsätzlich bekannt. So schlägt die WO 03/043 593 A1 vor, herkömmliche antibakterielle Substanzen wie Triclosan, Phenoxyethanol oder Hexetidin mit Ethyllauroylarginat zu kombinieren, um die antibakterielle Wirkung zu verstärken. In der WO 2007/014580 A1 werden Konservierungsmittelgemische vorgeschlagen, die neben Ethyllauroylarginat Salze von organischen oder anorganischen Säuren, insbesondere Natriumcitrat, Natriumacetat, Natriumglutamat, Natriumfumarat, Natriummalat, Natriumgluconat, Natriumlaurat, Natriumlactat, Natriumhexametaphosphat, Natrium-tert-Butyl-hydrochinat, Natriumpropylparabenat oder die Hydrochloride von Glucosamin oder Ethanolamin enthalten. Kosmetische Zusammensetzungen, die ein Konservierungsmittelgemisch aus Ethyllauroylarginat und Parabenen, Imidzolylharnstoff, Phenoxyethanol, DMDM Hydantoin, 2-Methyl-5-chlor-3,4-isothiazolinon/2-Methyl-3,4-isothiazolinon und Quaternium-15 enthalten, werden in der EP 1414394 B1 offenbart. Die Druckschrift WO-A-2005/011716 betrifft den Einsatz von prebiotisch wirksamen Pflanzenextrakten zur Förderung des Wachstums erwünschter Hautkeime.

Es besteht weiter der Bedarf, antimikrobielle Zusammensetzungen bereitzustellen, die hocheffektiv sind. Aufgabe der vorliegenden Erfindung ist es ferner, entsprechend hochwirksame wasserhaltige Emulsionen bereitzustellen, die stabil sind. Die Stabilität soll insbesondere in einem Temperaturintervall von 0°C bis 40°C, insbesondere von - 10,0 °C bis + 60°C vorliegen. Die Emulsion soll insbesondere über einen Zeitraum von mehreren Monaten, bevorzugt wenigstens 12 Wochen, stabil bleiben.
Eine weitere Aufgabe war insbesondere, wirksame Konservierungsmittel zu finden, die den Verzicht auf 4-Hydroxybenzoesäure und 4-Hydroxybenzoesäureester, so genannte "Parabene", ermöglichen.

Es wurde überraschender Weise gefunden, dass eine Emulsion enthaltend antibakterielle Wirkstoffe und eine spezielle Emulgatorkombination eine hervorragende Stabilität der Emulsion bewirkt. Ferner bleibt die erzielte Viskosität der Emulsion erhalten und ist stabil. Darüber hinaus besitzt die Emulsion einen hervorragenden Wirkungsgrad der antibakteriellen Wirkstoffe auf dem Substrat.

Ein erster Gegenstand der Erfindung ist eine kosmetische Zusammensetzung in Form einer Emulsion, enthaltend
a) mindestens einen antibakteriellen Wirkstoff,
   und
b) mindestens ein Salz von C₁₂₋₂₀-Alkylphosphat, insbesondere ein Salz von Cetylphosphat,
   und
c) mindestens ein C₁₄₋₂₀-Mono- oder Diacylglycerid, bevorzugt mindestens ein C₁₆₋₁₈-Mono- oder Diacylglycerid, besonders bevorzugt ausgewählt aus gehärteten Palmölglyceriden,
   und
d) mindestens ein Polymer, welches durch Polymerisation von zumindest 2-Methyl-2[(1-oxo-2-propenyl)amino]-1-propansulfonsäure (AMPS) erhalten wird
   und
e) Wasser.

Es ist erfindungsgemäß bevorzugt, wenn Wasser in der Emulsion in der kontinuierlichen Phase enthalten ist.

Bevorzugt liegen die erfindungsgemäßen kosmetischen Zusammensetzungen in Form einer flüs-sigen, fließfähigen oder festen Öl-in-Wasser-Emulsion, Wasser-in-Öl-Emulsion, Mehrfach-Emulsion, insbesondere einer Öl-in-Wasser-in-Öl- oder Wasser-in-Öl-in-Wasser-Emulsion, Makroemulsion, Miniemulsion, Mikroemulsion, PIT-Emulsion, Nanoemulsion, Pickering-Emulsion vor. Erfindungsgemäß besonders bevorzugte kosmetische Zusammensetzungen liegen in Form einer Öl-in-Wasser Emulsion vor.

Weiterhin ist es bevorzugt, wenn die kosmetische Zusammensetzung mindestens 20 Gew.-%, besonders bevorzugt mindestens 40 Gew.-%, ganz besonders bevorzugt mindestens 50 Gew.-% Wasser, insbesondere jeweils in der kontinuierlichen Phase, enthält.

Es hat sich als besonders bevorzugt herausgestellt, dass in der erfindungsgemäßen Zusammensetzung die Gesamtmenge der antibakteriellen Wirkstoffe jeweils bezogen auf das Gewicht der erfindungsgemäßen Zusammensetzung 0,5 Gew.-% bis 20,0 Gew.-%, besonders bevorzugt 2,0 Gew.-% bis 15,0 Gew.-%, ganz besonders bevorzugt 3,0 Gew.-% bis 10,0 Gew.-%, am bevorzugtesten 4,0 Gew.-% bis 9,0 Gew.-%, beträgt.

Es ist erfindungsgemäß bevorzugt den antibakteriellen Wirkstoff aus mindestens einer Verbindung der Gruppe auszuwählen, die gebildet wird aus
a. Diolen der Formel (I)

   HO-CH₂-CHR³-(CH₂)ₙ-R⁴ (I),

   in der R³ und R⁴ für -H oder -OH und n = für eine ganze Zahl von 1 bis 10 stehen, wobei R³ und R⁴ stets verschieden sind,
b. Salzen aromatischer Säuren der Formel (II) 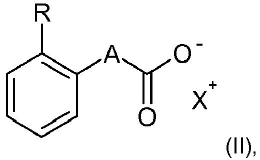 in der R für -H, -OH, ein Halogenatom oder eine C₁-C₄-Alkylgruppe, A für eine direkte Bindung oder Vinylen und X⁺ für ein Alkalimetallion oder den n-ten Teil eines n-wertigen Metallkations steht,
c. Dehydracetsäure (IIIa) und/oder deren Salze (IIIb) 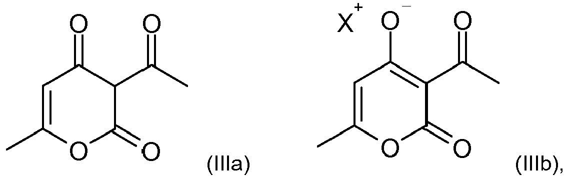 in der X⁺ für ein Alkalimetallion oder den n-ten Teil eines n-wertigen Metallkations steht,
d. Sorbinsäure und deren Salze,
e. 2-Phenoxyethanol,
f. Benzylalkohol, einzusetzen.

Geeignet ist bevorzugt mindestens ein Diol der Formel (I),

HO-CH₂CHR³-(CH₂)ₙ-R⁴ (I),

in der R³ und R⁴ für-H oder -OH und n = für eine ganze Zahl von 1 bis 10 stehen, wobei R³ und R⁴ stets verschieden sind. Es handelt sich dabei entweder um 1,2-Diole oder um alpha,omega-Diole mit 3 bis 12 Kohlenstoffatomen. Erfindungsgemäß bevorzugte Diole werden ausgewählt aus 1,2-Propandiol, 1,2-Butandiol, 1,2-Pentandiol, 1,2-Hexandiol, 1,2-Heptandiol, 1,2-Octandiol, 1,2-Nonandiol, 1,2-Decandiol, 1,2-Undecandiol, 1,2-Dodecandiol, 1,3-Propandiol, 1,4-Butandiol, 1,5-Pentandiol, 1,6-Hexandiol, 1,7-Heptandiol, 1,8-Octandiol, 1,9-Nonandiol, 1,10-Decandiol, 1,11-Undecandiol, 1,12-Dodecandiol sowie deren Mischungen.

Besonders bevorzugte erfindungsgemäße Zusammensetzungen enthalten als antibakteriellen Wirkstoff mindestens eine Verbindung aus der Gruppe
- 1,2-Propandiol (R³ = -OH, R⁴ = -H und n= 1 in Formel (I)) und/oder
- 1,3-Propandiol (R³= -H, R⁴= -OH und n= 1 in Formel (I)) und/oder
- 1,2-Hexandiol (R³= -OH, R⁴ = -H und n= 4 in Formel (I)) und/oder
- 1,6-Hexandiol (R³ = -H, R⁴ = -OH und n= 4 in Formel (I)) und/oder
- 1,2-Octandiol (R³ = -OH, R⁴ = -H und n= 6 in Formel (I)) und/oder
- 1,10-Decandiol (R³= -H, R⁴= -OH und n= 8 in Formel (I)).

Bevorzugt sind Diole der Formel (I) mit n = 4 sowie n = 6. Besonders bevorzugt sind Diole der Formel (I) mit n = 4, also 1,2-Hexandiol und 1,6-Hexandiol. Ein außerordentlich bevorzugtes Diol der Formel (I) ist 1,6-Hexandiol, welches auch ganz besonders bevorzugt als mindestens ein antibakterieller Wirkstoff in der erfindungsgemäßen Zusammensetzung enthalten ist.

Zusätzlich zu dem bzw. den Diol(en) der Formel (I) oder an ihrer Stelle können die erfindungsgemäßen Zusammensetzungen als antibakteriellen Wirkstoff mindestens eine Verbindung aus der Gruppe der Salze aromatischer Säuren der Formel (II) 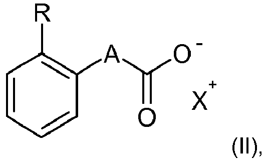 enthalten, in der R für -H, -OH, ein Halogenatom oder eine C₁-C₄-Alkylgruppe, A für eine direkte Bindung oder Vinylen und X⁺ für H₃O⁺, ein Alkalimetallion oder den n-ten Teil eines n-wertigen Metallkations steht.

Erfindungsgemäß geeignet sind beispielsweise die Natrium-, Kalium- oder Zinksalze von Säuren, in denen R für -H, -OH, -Cl, -Br, -CH₃, -CH₂CH₃, -CH(CH₃)₂, -(CH₂)₂CH₃, -C(CH₃)₃, -(CH₂)₃CH₃ steht.

Bevorzugte erfindungsgemäße Zusammensetzungen enthalten als antibakteriellen Wirkstoff mindestens eine Verbindung aus der Gruppe
- Natriumbenzoat (R = -H und A = direkte Bindung und X⁺ = Na+ in Formel (II)) und/oder
- Natriumsalicylat (R = -OH und A = direkte Bindung und X⁺ = Na+ in Formel (II)) und/oder
- Kaliumbenzoat (R = -H und A = direkte Bindung und X⁺ = K+ in Formel (II)) und/oder
- Kaliumsalicylat (R = -OH und A = direkte Bindung und X⁺ = K+ in Formel (II)) und/oder
- Natriumcinnamat (R = -H und A = Vinylen und X⁺ = Na+ in Formel (II)) und/oder
- Kaliumcinnamat (R = -H und A = Vinylen und X⁺ = K+ in Formel (II)) und/oder
- Zimtsäure (R = -H und A = Vinylen und X⁺ = H₃O+ in Formel (II)).

Zusätzlichen zu dem bzw. den Diol(en) der Formel (I) oder dem/den Salz(en) aromatischer Säuren der Formel (II) oder an ihrer Stelle können die erfindungsgemäßen Zusammensetzungen als antibakteriellen Wirkstoff mindestens eine Verbindung aus der Gruppe Dehydracetsäure (IIIa) und/oder deren Salze (IIIb) 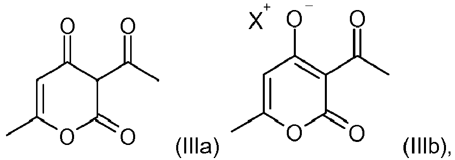 enthalten, in der X⁺ für ein Alkalimetallion oder den n-ten Teil eines n-wertigen Metallkations steht. Hier sind Dehydracetsäure, Kaliumdehydracetat und Natriumdehydracetat besonders bevorzugt.

Zusätzlichen zu dem bzw. den Diol(en) der Formel (I) oder dem/den Salz(en) aromatischer Säuren der Formel (II) und/oder mindestens einer (einem) Dehydracetsäure(salz) oder an ihrer Stelle können die erfindungsgemäßen Zusammensetzungen Sorbinsäure und/oder deren Salz enthalten. Wenn Sorbinsäure und/oder deren Salz enthalten ist, ist es wiederum bevorzugt, Natriumsorbat und/oder Kaliumsorbat zu verwenden.

Erfindungsgemäß wird der antibakterielle Wirkstoff besonders bevorzugt ausgewählt aus 1,2-Propandiol, 1,2-Butandiol, 1,2-Pentandiol, 1,2-Hexandiol, 1,2-Heptandiol, 1,2-Octandiol, 1,2-Nonandiol, 1,2-Decandiol, 1,2-Undecandiol, 1,2-Dodecandiol, 1,3-Propandiol, 1,4-Butandiol, 1,5-Pentandiol, 1,6-Hexandiol, 1,7-Heptandiol, 1,8-Octandiol, 1,9-Nonandiol, 1,10-Decandiol, 1,11-Undecandiol, 1,12-Dodecandiol, Benzoesäure, Salicylsäure, Zimtsäure, Natriumbenzoat, Natriumsalicylat, Kaliumbenzoat, Kaliumsalicylat, Natriumcinnamat, Kaliumcinnamat, Dehydracetsäure, Kaliumdehydracetat, Natriumdehydracetat, Natriumsorbat, Kaliumsorbat, Sorbinsäure, 2-Phenoxyethanol, Benzylalkohol, sowie aus Mischungen von mindestens zwei der vorgenannten Verbindungen.

Als besonders bevorzugte Kombination antibakterieller Wirkstoffe enthält die erfindungsgemäße Zusammensetzung bevorzugt zumindest
i) mindestens ein Diol der Formel (I)

   HO-CH₂-CHR³-(CH₂)ₙ-R⁴ (I),

   in der R³ und R⁴ für -H oder -OH und n = für eine ganze Zahl von 1 bis 10 stehen, wobei R³ und R⁴ stets verschieden sind (insbesondere zumindest 1,6-Hexandiol)
   und
ii) mindestens ein Salz aromatischer Säuren der Formel (II) 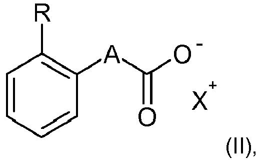 in der R für -H, -OH, ein Halogenatom oder eine C₁-C₄-Alkylgruppe, A für eine direkte Bindung oder Vinylen und X⁺ für ein Alkalimetallion oder den n-ten Teil eines n-wertigen Metallkations steht,
   und besonders bevorzugt zusätzlich
iii) 2-Phenoxyethanol.

Als besonders bevorzugte Kombination antibakterieller Wirkstoffe enthält die erfindungsgemäße Zusammensetzung jeweils bezogen auf das Gesamtgewicht der Zusammensetzung bevorzugt zumindest
i) 0,5 Gew.-% bis 10,0 Gew.-%, insbesondere 2,0 Gew.-% bis 8,0 Gew.-% mindestens eines Diols der Formel (I)

   HO-CH₂-CHR₃-(CH₂)ₙ-R⁴ (I),

   in der R³ und R⁴ für -H oder -OH und n = für eine ganze Zahl von 1 bis 10 stehen, wobei R³ und R⁴ stets verschieden sind (insbesondere zumindest 1,6-Hexandiol)
   und
ii) 0,05 bis 2,0 Gew.-%, insbesondere 0,1 bis 0,5 Gew.-%, mindestens ein Salz aromatischer Säuren der Formel (II) 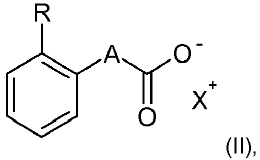 in der R für -H, -OH, ein Halogenatom oder eine C₁-C₄-Alkylgruppe, A für eine direkte Bindung oder Vinylen und X⁺ für ein Alkalimetallion oder den n-ten Teil eines n-wertigen Metallkations steht,
   und besonders bevorzugt zusätzlich
iii) 0,1 bis 5,0 Gew.-%, insbesondere 0,5 bis 3,0 Gew.-%, 2-Phenoxyethanol.

Die erfindungsgemäße kosmetische Zusammensetzung enthält zwingend mindestens ein Salz von C₁₂₋₂₀-Alkylphosphaten, insbesondere mindestens ein Salz des Cetylphosphats.

Salze von C₁₂₋₂₀-Alkylphosphaten, umfassen Phosphorsäure-C₁₂₋₂₀-Monoalkylester und Phosphorsäure-C₁₂₋₂₀-Dialkylester.

Die Salze von C₁₂₋₂₀-Alkylphosphaten können bespielsweise als Alkali-, Erdalkali-, Ammonium-, Alkylammonium-, Alkanolamin- oder Glucammoniumsalz vorliegen. Bevorzugt sind die entsprechenden Natrium-, Kalium-, Alkanolamin-, Trialkylammonium-, Triethanolamin-, 2-Amino-1-butanol-, 2-Amino-2-methyl-1-propanol-, 2-Amino-2-methyl-1,3-propandiol-, 2-Amino-2-ethyl-1,3-propandiol- oder Tris-(hydroxymethyl)aminomethan-Salze, sowie Salze der basischen Aminosäuren Ornithin, Lysin, Arginin und/oder Histidin. Besonders bevorzugt sind die Kaliumsalze besagter C₁₂₋₂₀-Alkylphosphate.

Erfindungsgemäß bevorzugte kosmetische Zusammensetzungen sind dadurch gekennzeichnet, dass die Salze von C₁₂₋₂₀-Alkylphosphat, insbesondere die Salze von Cetylphosphat,, in einer Gesamtmenge von 0,2 bis 2,0 Gew.-%, bevorzugt 0,3 bis 1,8 Gew.-%, besonders bevorzugt 0,3 bis 0,7 Gew.-%, jeweils bezogen auf das Gesamtgewicht der Zusammensetzung, enthalten sind.

Erfindungsgemäß bevorzugte Salze von C₁₂₋₂₀-Alkylphosphaten, sind ausgewählt aus den Monoestern von Phosphorsäure mit Laurylalkohol, Tridecylalkohol, Isotridecylalkohol, Myristylalkohol, Pentadecylalkohol, Cetylalkohol, Palmitylalkohol, Isocetylalkohol, Isostearylalkohol, Stearylalkohol, Oleylalkohol, Elaidylalkohol, Linoleylalkohol, Linolenylalkohol, Nonadecylalkohol, Arachylalkohol, Gadoleylalkohol oder Arachidonalkohol, die als Alkali-, Erdalkali-, Ammonium-, Alkylammonium-, Alkanolamin- oder Glucammoniumsalz, bevorzugt die entsprechenden Natrium-, Kalium-, Alkanolamin-, Trialkylammonium-, Triethanolamin-, 2-Amino-1-butanol-, 2-Amino-2-methyl-1-propanol-, 2-Amino-2-methyl-1,3-propandiol-, 2-Amino-2-ethyl-1,3-propandiol- oder Tris-(hydroxymethyl)aminomethan-Salze sowie als Salze der basischen Aminosäuren Ornithin, Lysin, Arginin und/oder Histidin, vorliegen. Bevorzugt sind die Kaliumsalze der genannten Phosphorsäuremonoester. Besonders bevorzugt ist Dikaliummonocetylphosphat.
Weitere erfindungsgemäß bevorzugte Salze von C₁₂₋₂₀-Alkylphosphaten sind ausgewählt aus den Diestern von Phosphorsäure mit Laurylalkohol, Tridecylalkohol, Isotridecylalkohol, Myristylalkohol, Pentadecylalkohol, Cetylalkohol, Palmitylalkohol, Isocetylalkohol, Isostearylalkohol, Stearylalkohol, Oleylalkohol, Elaidylalkohol, Linoleylalkohol, Linolenylalkohol, Nonadecylalkohol, Arachylalkohol, Gadoleylalkohol oder Arachidonalkohol, die als Alkali-, Erdalkali-, Ammonium-, Alkylammonium-, Alkanolamin- oder Glucammoniumsalz, bevorzugt die entsprechenden Natrium-, Kalium-, Alkanolamin-, Trialkylammonium-, Triethanolamin-, 2-Amino-1-butanol-, 2-Amino-2-methyl-1-propanol-, 2-Amino-2-methyl-1,3-propandiol-, 2-Amino-2-ethyl-1,3-propandiol- oder Tris-(hydroxymethyl)aminomethan-Salze sowie als Salze der basischen Aminosäuren Ornithin, Lysin, Arginin und/oder Histidin, vorliegen. Bevorzugt sind die Kaliumsalze der genannten Phosphorsäurediester. Besonders bevorzugt ist Kaliumdicetylphosphat.

Besonders bevorzugt sind Mischungen aus Mono-C₁₂₋₂₀-Alkylphosphaten und Di-C₁₂₋₂₀-Alkylphos-phaten. Außerordentlich bevorzugt sind Mischungen aus Dikaliummonocetylphosphat und Kaliumdicetylphosphat.

Die erfindungsgemäße kosmetische Zusammensetzung enthält zwingend mindestens ein C₁₄₋₂₀-Mono- oder Diacylglycerid, bevorzugt mindestens ein C₁₆₋₁₈-Mono- oder Diacylglycerid. Diese werden jeweils besonders bevorzugt aus gehärteten Palmölglyceriden ausgewählt. Sie tragen die INCl-Deklaration Hydrogenated Palm Glycerides.

Erfindungsgemäß bevorzugte C₁₄₋₂₀-Mono- oder Diacylglyceride sind ausgewählt aus Monomyristoylglycerid, Monopalmitoylglycerid, Monostearoylglycerid, Monoarachinoylglycerid, Dimyristoylglycerid, Dipalmitoylglycerid, Distearoylglycerid, Diarachinoylglycerid sowie Mischungen zweier oder meherer Verbindungen daraus. Weitere erfindungsgemäß bevorzugte C₁₄₋₂₀-Mono-oder Diacylglyceride sind Glyceride gehärteter, das heißt, hydrierter, bevorzugt vollständig hydrierter, Fettsäuren natürlicher Öle. Erfindungsgemäß besonders bevorzugt sind gehärtete Palmölglyceride.

Erfindungsgemäß bevorzugte kosmetische Zusammensetzungen enthalten die C₁₄₋₂₀-Mono- oder Diacylglyceride, bevorzugt die C₁₆₋₁₈-Mono- oder Diacylglyceride, besonders bevorzugt die gehärteten Palmölglyceride, in einer Gesamtmenge von 0,1 bis 1,7 Gew.-% bezogen auf das Gesamtgewicht der Zusammensetzung.

Der erfindungsgemäße Effekt wird besonders ausgeprägt, wenn die Salze von C₁₂₋₂₀-Alkylphosphat (insbesondere die Salze von Cetylphosphat) und die C₁₄₋₂₀-Mono- und C₁₄₋₂₀-Diacylglyceride bevorzugt in einem Gewichtsverhältnis C₁₂₋₂₀-Alkylphosphat-Salz zu besagtem Glycerid von 3 zu 1 bis 1 zu 1,5, besonders bevorzugt von 2,5 zu 1 bis 1 zu 1, in der erfindungsgemäßen Zusammensetzung enthalten sind.

Es ist erfindungsgemäß besonders bevorzugt, wenn die erfindungsgemäße kosmetische Zusammensetzung
- die Salze von C₁₂₋₂₀-Alkylphosphat (insbesondere die Salze von Cetylphosphat) und die C₁₄₋₂₀-Mono- und C₁₄₋₂₀-Diacylglyceride in einem Bereich des Gewichtsverhältnisses C₁₂₋₂₀-Alkylphosphat-Salz zu besagtem Glycerid von 3 zu 1 bis 1 zu 1,5, bevorzugt von 2,5 zu 1 bis 1 zu 1, und
- die Salze von C₁₂₋₂₀-Alkylphosphat, insbesondere die Salze von Cetylphosphat in einer Gesamtmenge von 0,2 bis 2,0 Gew.-%, bevorzugt 0,3 bis 1,8 Gew.-%, besonders bevorzugt 0,3 bis 0,7 Gew.-%, jeweils bezogen auf das Gesamtgewicht der Zusammensetzung, enthalten, und
- die C₁₆₋₁₈-Mono- oder Diacylglyceride, besonders bevorzugt die gehärteten Palmölglyceride, in einer Gesamtmenge von 0,1 bis 1,7 Gew.-% bezogen auf das Gesamtgewicht der Zusammensetzung,
enthält.

Die erfindungsgemäßen kosmetischen Zusammensetzungen enthalten zwingend mindestens ein Polymer, welches durch Polymerisation von zumindest 2-Methyl-2[(1-oxo-2-propenyl)amino]-1-propansulfonsäure (AMPS) erhalten wird.

Die erfindungsgemäße kosmetische Zusammensetzung enthält besagte Polymere bevorzugt in einer Gesamtmenge von 0,005 bis 5,0 Gew.-%, insbesondere 0,005 - 2,5 Gew.-%, besonders bevorzugt 0,1 bis 2 Gew.-%, jeweils bezogen auf das Gewicht der Zusammensetzung.

Die erfindungsgemäßen kosmetischen Mittel dieser Ausführungsform enthalten bevorzugt als besagtes Polymer mindestens ein Homopolymer oder Copolymer der 2-Methyl-2[(1-oxo-2-propenyl)amino]-1-propansulfonsäure (AMPS), dessen Säuregruppen teilweise oder vollständig neutralisiert sind. Dieses Polymer ist wiederum bevorzugt vernetzt.

Aus der partiellen Neutralisation der Säuregruppen resultieren der neutralisierte Monomerbaustein, beispielsweise Natrium-2-methyl-2[(1-oxo-2-propenyl)amino]-1-propansulfonat oder Ammonium-2-methyl-2[(1-oxo-2-propenyl)amino]-1-propansulfonat, und der nicht-neutralisierte, saure Monomerbaustein, 2-Methyl-2[(1-oxo-2-propenyl)amino]-1-propansulfonsäure. Als neutralisiertes Monomer geeignet sind alle 2-Methyl-2[(1-oxo-2-propenyl)amino]-1-propansulfonatsalze. Bevorzugt sind das Natrium-, Kalium-, Ammonium-, Ethanolamin- oder Aminosäure-Salz. Besonders bevorzugt sind Natrium-2-methyl-2[(1-oxo-2-propenyl)amino]-1-propansulfonat und Ammonium-2-methyl-2[(1-oxo-2-propenyl)amino]-1-propansulfonat.

Ein erstes erfindungsgemäß bevorzugtes Polymer ist das vernetzte AMPS-Homopolymer Ammonium Polyacryloyldimethyl Taurate. Ein derartiges vernetztes AMPS-Homopolymer ist beispielsweise von Clariant unter der Bezeichnung Hostacerin AMPS erhältlich.

Besonders bevorzugt sind neben den (bevorzugt vernetzten) AMPS-Homopolymeren die (insbesondere vernetzten) AMPS-Copolymere. Bevorzugte solcher AMPS-Copolymere enthalten neben AMPS ein, zwei oder drei, verschiedene Monomere, die neutral sein oder eine schwache Säurefunktion aufweisen können. Selbstverständlich sind auch AMPS-Copolymere geeignet, die neben AMPS vier oder sogar mehr Monomere aufweisen.

In einer weiteren bevorzugten Ausführungsform der vorliegenden Erfindung enthält das (bevorzugt vernetzte) AMPS-Copolymer als zweites Monomer ein neutrales Monomer, das ausgewählt ist aus 2-Hydroxyethylacrylat, 2,3-Dihydroxypropylacrylat, 2-Hydroxyethylmethacrylat, 2,3-Dihydroxy-propylmethacrylat, Acrylamid, Alkylacrylamiden, wie beispielsweise Methylacrylamid, Ethylacrylamid, n-Propylacrylamid und Isopropylacrylamid, Dialkylamiden, wie beispielsweise Dimethylacrylamid, Diethylacrylamid, Di-n-propylacrylamid und Diisopropylacrylamid, den ethoxylierten Derivaten der vorstehend genannten Ester und Amide, sowie N-Vinylpyrrolidon. Besonders bevorzugt ist das neutrale Polyelektrolytmonomer ausgewählt aus 2-Hydroxy-ethylacrylat, 2-Hydroxyethylmethacrylat, 2,3-Dihydroxypropylmethacrylat, den ethoxylierten Derivaten der genannten Ester, Acrylamid, Dimethylacrylamid, sowie N-Vinylpyrrolidon. Außerordentlich bevorzugte neutrale Polyelektrolytmonomere sind ausgewählt aus 2-Hydroxy-ethylacrylat, Acrylamid, Dimethylacrylamid und Vinylpyrrolidon. Außerordentlich bevorzugte AMPS-Copolymere sind ausgewählt aus vernetzten Copolymeren, bestehend aus AMPS und N-Vinylpyrrolidon, AMPS und 2-Hydroxyethylacrylat, AMPS und Acrylamid, AMPS und Dimethylacrylamid.

Besonders bevorzugte ethoxylierte Derivate der vorstehend genannten (Meth)Acrylsäureester und (Meth)Acrylsäureamide sind solche der nachstehenden Formel (AMPS-Copol), in denen R1 und R3, die gleich oder verschieden sind, ein Wasserstoffatom oder eine in etwa lineare oder verzweigte Alkylgruppe mit 1 bis 6 Kohlenstoffatomen bedeuten; Y O oder NH bedeutet; R2 eine Kohlenwasserstoffgruppe mit 6 bis 50 Kohlenstoffatomen ist; und x die Molzahl des Alkylenoxids angibt und im Bereich von 1 bis 50, bevorzugt 8 bis 20, liegt: 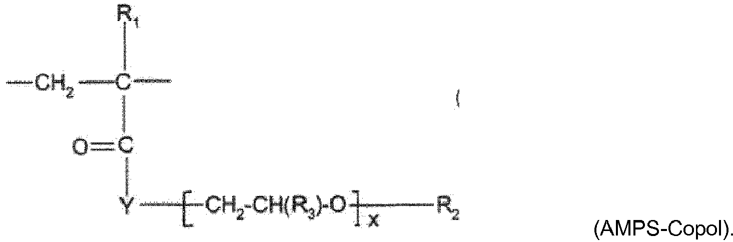 Ein bevorzugtes vernetztes Ammonium Acryloyldimethyltaurate/VP Copolymer ist von Clariant unter der Bezeichnung Aristoflex AVC erhältlich.

In einer weiteren ganz besonders bevorzugten Ausführungsform der vorliegenden Erfindung wird das (bevorzugt vernetzte) Polymer durch Copolymerisation von zumindest AMPS mit mindestens einem Monomeren mit schwacher Säurefunktion, das ausgewählt ist aus Acrylsäure, Methacrylsäure, Itaconsäure und Maleinsäure, erhalten. Dabei ist es wiederum bevorzugt, wenn die schwache Säurefunktion teilweise oder vollständig neutralisiert ist und als Salz, bevorzugt als Natrium-, Kalium-, Ammonium-, Ethanolamin- oder Aminosäure-Salz, besonders bevorzugt als Natriumsalz, vorliegt. Weitere außerordentlich bevorzugte vernetzte AMPS-Copolymere sind ausgewählt aus vernetzten Copolymeren, bestehend aus AMPS und Natriumacrylat, AMPS und Natriummethacrylat, AMPS und Acrylamid und Natriumacrylat, AMPS und Acrylamid und Natriumacrylat und Acrylsäure.

Ein ganz besonders bevorzugtes vernetztes Sodium Acrylate/Sodium Acryloyldimethyl Taurate Copolymer ist von Seppic unter der Bezeichnung Simulgel EPG erhältlich.
Weitere bevorzugte vernetzte Copolymere mit 2-Methyl-2[(1-oxo-2-propenyl)amino]-1-propansulfonsäure (AMPS) sind kommerziell erhältlich, z. B. unter den Handelsnamen Simulgel^{®}NS, Simulgel^{®}NS 100, Simulgel^{®}FL, Sepiplus^{®} S, Simulgel^{®}600, Sepiplus 400, Simulgel^{®}SMS 88, Simulgel^{®}EG und Simulgel^{®}EPG von der Firma Seppic.

Es sind weitere erfindungsgemäß bevorzugte Zusammensetzungen dadurch gekennzeichnet, dass sie zusätzlich mindestens einen konditionierenden Wirkstoff enthalten. Unter konditionierenden Wirkstoffen sind erfindungsgemäß solche Substanzen zu verstehen, die auf keratinische Materialien, insbesondere auf die Haut, aufziehen und die physikalischen und sensorischen Eigenschaften sowohl der Haut als auch des Produktes als solchem verbessern. Konditionierungsmittel glätten die oberste Schicht der Haut und machen sie weich und geschmeidig. Über die Auswahl der Konditionierungsmittel (fettig - weniger fettig, schnell oder langsam spreitend, schnell oder langsam in die Haut einziehend und so weiter) lässt sich das Hautgefühl des gesamten Produktes einstellen.

Die erfindungsgemäßen Zusammensetzungen enthalten bevorzugt mindestens einen Fettkörper. Als Fettkörper besonders bevorzugt mindestens einen Fettstoff. Unter sind Fettsäuren, Fettalkohole, natürliche und synthetische Wachse und natürliche und synthetische kosmetische Ölkomponenten zu verstehen.

Die Fettstoffe sind in den erfindungsgemäßen Zusammensetzungen bevorzugt in einer Menge von 5,0 bis 50,0 Gew.-%, besonders bevorzugt von 8,0 bis 30,0 Gew.-%, jeweils bezogen auf das Gesamtgewicht des Zusammensetzung, enthalten.

Als Fettstoff eignen sich bevorzugt lineare, gesättigte primäre Alkohole mit 14 - 30 Kohlenstoffatomen, Fettsäuren mit 6 bis 30 Kohlenstoffatomen, Triglyzeride, Wachse, Fettsäureester, natürliche Öle, Silikone.

Als eine Komponente des Fettkörpers enthalten die erfindungsgemäßen Zusammensetzungen bevorzugt mindestens einen linearen, gesättigten primären Alkohol mit 14 - 30 Kohlenstoffatomen.

Erfindungsgemäß bevorzugte lineare, gesättigte primäre Alkohole mit 14 - 30 Kohlenstoffatomen sind ausgewählt aus Myristylalkohol, Cetylalkohol, Stearylalkohol, 12-Hydroxystearylalkohol, Arachidylalkohol (Arachylalkohol), Behenylalkohol, Lignocerylalkohol, Cerylalkohol und Myricylalkohol und Mischungen hiervon, insbesondere technische Mischungen wie Arachidylalkohol und Behenylalkohol sowie Cetylalkohol und Stearylalkohol (Cetearylalkohol). Besonders bevorzugt sind Cetylalkohol, Stearylalkohol, Arachidylalkohol und Behenylalkohol und Mischungen hiervon. Besonders bevorzugt sind Mischungen aus Cetearylalkohol und Behenylalkohol.

Die vorgenannten Alkohole werden im Folgenden auch synonym als "Fettalkohole" bezeichnet.

Bevorzugte kosmetische Zusammensetzungen enthalten mindestens einen linearen, gesättigten primären Alkohol mit 14 - 30 Kohlenstoffatomen in einer Gesamtmenge von 0,5 bis 10,0 Gew.-%, bevorzugt 1,0 bis 8,0 Gew.-%, jeweils bezogen auf das Gesamtgewicht der Zusammensetzung.

Als weitere Fettstoffe können die erfindungsgemäßen Zusammensetzungen bevorzugt mindestens eine Fettsäure mit 6 bis 30 Kohlenstoffatomen enthalten.

Als Fettsäuren können eingesetzt werden lineare und/oder verzweigte, gesättigte und/oder ungesättigte Fettsäuren mit 6 - 30 Kohlenstoffatomen. Bevorzugt sind Fettsäuren mit 10 - 22 Kohlenstoffatomen. Hierunter wären beispielsweise zu nennen die Isostearinsäuren, wie die Handelsprodukte Emersol^{®}871 und Emersol^{®} 875, und Isopalmitinsäuren wie das Handelsprodukt Edenor^{®} IP 95, sowie alle weiteren unter den Handelsbezeichnungen Edenor^{®} (Cognis) vertriebenen Fettsäuren. Weitere typische Beispiele für solche Fettsäuren sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Oxidation von Aldehyden aus der Roelen'schen Oxosynthese oder der Dimerisierung von ungesättigten Fettsäuren anfallen. Besonders bevorzugt sind üblicherweise die Fettsäureschnitte, welche aus Cocosöl oder Palmöl erhältlich sind; insbesondere bevorzugt ist in der Regel der Einsatz von Stearinsäure.

Die Einsatzmenge an Fettsäure beträgt dabei bevorzugt 0,1 bis 15 Gew.%, bezogen auf das gesamte Zusammensetzung. In einer besonders bevorzugten Ausführungsform beträgt die Menge 0,5 bis 10 Gew.%, wobei ganz besonders vorteilhaft Mengen von 1 bis 5 Gew.% sind.

Als natürliche oder synthetische Wachse können erfindungsgemäß eingesetzt werden feste Paraffine oder Isoparaffine, Carnaubawachse, Bienenwachse, Candelillawachse, Ozokerite, Ceresin, Walrat, Sonnenblumenwachs, Fruchtwachse wie beispielsweise Apfelwachs oder Citruswachs, Microwachse aus PE- oder PP. Derartige Wachse sind beispielsweise erhältlich über die Fa. Kahl & Co., Trittau.

Zu den natürlichen und synthetischen kosmetischen Ölkörpern, eignen sich bevorzugt:
- pflanzliche Öle. Beispiele für solche Öle sind Sonnenblumenöl, Olivenöl, Sojaöl, Rapsöl, Mandelöl, Jojobaöl, Orangenöl, Weizenkeimöl, Pfirsichkernöl und die flüssigen Anteile des Kokosöls. Geeignet sind aber auch andere Triglyceridöle wie die flüssigen Anteile des Rindertalgs sowie synthetische Triglyceridöle, insbesondere Capryl-/Caprinsäuretriglyzerid.
- flüssige Paraffinöle, Isoparaffinöle und synthetische Kohlenwasserstoffe sowie Di-n-alkylether mit insgesamt zwischen 12 bis 36 C-Atomen, insbesondere 12 bis 24 C-Atomen, wie beispielsweise Di-n-octylether, Di-n-decylether, Di-n-nonylether, Di-n-undecylether, Di-n-dodecylether, n-Hexyl-n-octylether, n-Octyl-n-decylether, n-Decyl-n-undecylether, n-Undecyl-n-dodecylether und n-Hexyl-n-Undecylether sowie Di-tert-butylether, Di-iso-pentylether, Di-3-ethyldecylether, tert.-Butyl-n-octylether, iso-Pentyl-n-octylether und 2-Methyl-pentyl-n-octylether. Die als Handelsprodukte erhältlichen Verbindungen 1,3-Di-(2-ethyl-hexyl)-cyclohexan (Cetiol^{®} S) und Di-n-octylether (Cetiol^{®} OE) können bevorzugt sein.
- Esteröle. Unter Esterölen sind zu verstehen die Ester von C₆-C₃₀ - Fettsäuren mit C₂-C₃₀-Fettalkoholen. Bevorzugt sind die Monoester der Fettsäuren mit Alkoholen mit 2 bis 24 C-Atomen. Beispiele für eingesetzte Fettsäurenanteile in den Estern sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Oxidation von Aldehyden aus der Roelen'schen Oxosynthese oder der Dimerisierung von ungesättigten Fettsäuren anfallen. Beispiele für die Fettalkoholanteile in den Esterölen sind Isopropylalkohol, Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen, die z.B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen. Erfindungsgemäß besonders bevorzugt sind Isopropylmyristat (Rilanit^{®} IPM), Isononansäure-C16-18-alkylester (Cetiol^{®} SN), 2-Ethylhexylpalmitat (Cegesoft^{®} 24), Stearinsäure-2-ethylhexylester (Cetiol^{®} 868), Cetyloleat, Glycerintricaprylat, Kokosfettalkohol-caprinat/-caprylat (Cetiol^{®} LC), n-Butylstearat, Oleylerucat (Cetiol^{®} J 600), Isopropylpalmitat (Rilanit^{®} IPP), Oleyl Oleate (Cetiol^{®}), Laurinsäurehexylester (Cetiol^{®} A), Di-n-butyladipat (Cetiol^{®} B), Myristylmyristat (Cetiol^{®} MM), Cetearyl Isononanoate (Cetiol^{®} SN), Ölsäuredecylester (Cetiol^{®} V).
- Dicarbonsäureester wie Di-n-butyladipat, Di-(2-ethylhexyl)-adipat, Di-(2-ethylhexyl)-succinat und Di-isotridecylacelaat sowie Diolester wie Ethylenglykol-dioleat, Ethylenglykol-diisotridecanoat, Propylenglykol-di(2-ethylhexanoat), Propylenglykol-di-isostearat, Propylenglykol-di-pelargonat, Butandiol-di-isostearat, Neopentylglykoldicaprylat,
- symmetrische, unsymmetrische oder cyclische Ester der Kohlensäure mit Fettalkoholen, beispielsweise beschrieben in der DE-OS 197 56 454, Glycerincarbonat oder Dicaprylylcarbonat (Cetiol^{®} CC),
- Trifettsäureester von gesättigten und/oder ungesättigten linearen und/oder verzweigten Fettsäuren mit Glycerin,
- Triglyceriden gesättigter und gegebenenfalls hydroxylierter C₁₆₋₃₀-Fettsäuren, z. B. gehärteten Triglyceridfetten, insbesondere Triglyceride aus gehärtetem Palmöl.
- Phospholipiden, beispielsweise Sojalecithin, Ei-Lecithin und Kephalinen,
- Siliconverbindungen, ausgewählt aus Decamethylcyclopentasiloxan, Dodecamethylcyclohexasiloxan und Siliconpolymeren, die gewünschtenfalls quervernetzt sein können, z. B. Polydialkylsiloxanen, Polyalkylarylsiloxanen, ethoxylierten und/oder propoxylierten Polydialkylsiloxanen mit der früheren INCl-Bezeichnung Dimethicone Copolyol, sowie Polydialkylsiloxanen, die Amin- und/ oder Hydroxy-Gruppen enthalten, bevorzugt Substanzen mit den INCI-Bezeichnungen Dimethiconol, Amodimethicone oder Trimethylsilylamodimethicone.

Die Einsatzmenge der Fettstoffe beträgt bevorzugt 0,1 - 99 Gew.-%, besonders bevorzugt 2 - 50 Gew.-% und außerordentlich bevorzugt 5 - 20 Gew.-%, jeweils bezogen auf die gesamte erfindungsgemäße Zusammensetzung.

Weitere mögliche Inhaltsstoffe der erfindungsgemäßen kosmetischen Zusammensetzungen können über ihre Funktion definiert werden. Natürlich können manche Inhaltsstoffe auch multifunktionell sein. Bevorzugte Inhaltsstoffe der erfindungsgemäßen kosmetischen Zusammensetzungen können sein: Absorptionsmittel, antimikrobielle Stoffe, Bindemittel, Bleichmittel, Chelatbildner, Emollientien, Enthaarungsmittel und Wirkstoffe mit Haarwuchs inhibierender Wirkung, Feuchtigkeitsspender, Filmbildner, Farbstoffe, Konservierungsstoffe, Korrosionsschutzmittel, hautkühlende Wirkstoffe, pH-Wert-Regler/ Puffersubstanzen, Treibgase, Trübungsmittel, UV-Absorber/ Lichtfiltersubstanzen, Vergällungsmittel. Ferner können auch Kombinationen der vorgenannten Inhaltsstoffe in den erfindungsgemäß bevorzugten Zusammensetzungen enthalten sein.

Ein zweiter Gegenstand der Erfindung ist die Verwendung der Zusammensetzungen des ersten Erfindungsgegenstandes zur Pflege der Haut, insbesondere zur Pflege von Altershaut.

Ein dritter Gegenstand der Erfindung ist ein Verfahren zur Hautpflege, in dem eine Zusammensetzung des ersten Erfindungsgegenstandes auf die Haut aufgetragen und auf der Haut für einen Zeitraum von mindestens einer Stunde belassen wird.

### Beispiele

Es wurden folgende Emulsionen als Hautpflegemittel nach einem Standard Emulgierverfahren unter Einsatz der erfindungsgemäßen Inhaltsstoffe hergestellt:

| **Rohstoff** | **E1** **[Gew.-%]** | **E2** **[Gew.-%]** |
|---|---|---|
| Emulsiphos 677660 ¹ | 1,00 | 1,00 |
| Capryl-/Caprinsäure-Triglycerid | 3,50 | 3,50 |
| Cutina MD ² | 2,00 | 2,00 |
| Cetearyl Alcohol | 1,00 | 1,00 |
| Dimethicone (200-300 cSt) | 0,50 | 0,50 |
| Vitamin E Acetate | 0,50 | 0,50 |
| 1,6-Hexandiol | 5,00 | 5,00 |
| Glyzerin | 2,00 | 2,00 |
| Sorbitol | 0,50 | 0,80 |
| Carbomer | 0,30 | 0,30 |
| D-Panthenol | 0,60 | 0,60 |
| 2-Phenoxyethanol | 0,90 | 1,00 |
| D,L-alpha-Bisabolol | 0,10 | 0,10 |
| Natriumsalicylat | 0,40 | - |
| Natriumbenzoat | - | 0,40 |
| Acnacidol BG ³ | 0,50 | 0,50 |
| Aluminium Starch Octenylsuccinate | 1,00 | 1,00 |
| Simulgel EPG ⁴ | 1,00 | 1,00 |
| Natriumhydroxid | 0,04 | 0,04 |
| Parfum | 0,20 | |
| Wasser | ad 100 | ad 100 |

| | | |
|---|---|---|
| 1 INCl-Bezeichnung: Potassium Cetyl Phosphate, Hydrogenated Palm Glycerides (Symrise) 2 Glycerinmono/distearat (INCl-Bezeichnung: Glyceryl Stearate) (BASF SE) 3 INCl-Bezeichnung: Butylene Glycol, 10-Hydroxydecanoic Acid, Sebacic Acid, 1,10-Decanediol (ISP) 4 INCl-Bezeichnung: SODIUM ACRYLATE/SODIUM ACRYLOYLDIMETHYL TAURATE COPOLYMER, POLYISOBUTENE, CAPRYLYL/CAPRYL GLUCOSIDE (Seppic) | | |

Die Zusammensetzungen wiesen eine gute Lagerstabilität auf. Sie ließen sich auf der Haut leicht verteilen und zeigten eine antibakterielle Wirkung.

## Patentansprüche

1. Kosmetische Zusammensetzung in Form einer Emulsion, enthaltend
a) mindestens einen antibakteriellen Wirkstoff,
und
b) mindestens ein Salz von C₁₂₋₂₀-Alkylphosphat, insbesondere ein Salz von Cetylphosphat, und
c) mindestens ein C₁₄₋₂₀-Mono- oder Diacylglycerid, bevorzugt mindestens ein C₁₆₋₁₈-Mono-oder Diacylglycerid, besonders bevorzugt ausgewählt aus gehärteten Palmölglyceriden, und
d) mindestens ein Polymer, welches durch Polymerisation von zumindest 2-Methyl-2[(1-oxo-2-propenyl)amino]-1-propansulfonsäure (AMPS) erhalten wird
und
e) Wasser.

2. Kosmetische Zusammensetzung gemäß Anspruch 1 **dadurch gekennzeichnet, dass** die Gesamtmenge der antibakteriellen Wirkstoffe jeweils bezogen auf das Gewicht der erfindungsgemäßen Zusammensetzung 0,5 Gew.-% bis 20,0 Gew.-%, besonders bevorzugt 2,0 Gew.-% bis 15,0 Gew.-%, ganz besonders bevorzugt 3,0 Gew.-% bis 10,0 Gew.-%, am bevorzugtesten 4,0 Gew.-% bis 9,0 Gew.-%, beträgt.

3. Kosmetische Zusammensetzung gemäß Anspruch 1 oder 2, **dadurch gekennzeichnet, dass** der antibakterielle Wirkstoff aus mindestens einer Verbindung der Gruppe ausgewählt wird, die gebildet wird aus
a. Diolen der Formel (I)
HO-CH₂-CHR³-(CH₂)ₙ-R⁴ (I),
in der R³ und R⁴ für -H oder -OH und n = für eine ganze Zahl von 1 bis 10 stehen, wobei R³ und R⁴ stets verschieden sind,
b. Salzen aromatischer Säuren der Formel (II) 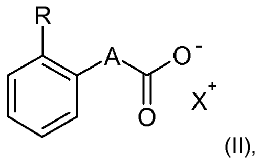 in der R für -H, -OH, ein Halogenatom oder eine C₁-C₄-Alkylgruppe, A für eine direkte Bindung oder Vinylen und X⁺ für ein Alkalimetallion oder den n-ten Teil eines n-wertigen Metallkations steht,
c. Dehydracetsäure (IIIa) und/oder deren Salze (IIIb) 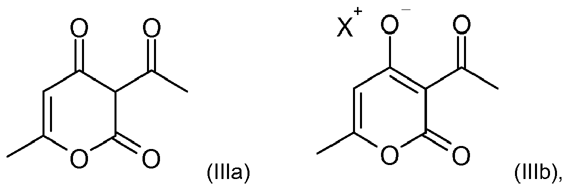 in der X⁺ für ein Alkalimetallion oder den n-ten Teil eines n-wertigen Metallkations steht,
d. Sorbinsäure und deren Salze,
e. 2-Phenoxyethanol,
f. Benzylalkohol.

4. Kosmetische Zusammensetzung gemäß einem der Ansprüche 1 bis 3, **dadurch gekennzeichnet, dass** der antibakterielle Wirkstoff ausgewählt wird aus 1,2-Propandiol, 1,2-Butandiol, 1,2-Pentandiol, 1,2-Hexandiol, 1,2-Heptandiol, 1,2-Octandiol, 1,2-Nonandiol, 1,2-Decandiol, 1,2-Undecandiol, 1,2-Dodecandiol, 1,3-Propandiol, 1,4-Butandiol, 1,5-Pentandiol, 1,6-Hexandiol, 1,7-Heptandiol, 1,8-Octandiol, 1,9-Nonandiol, 1,10-Decandiol, 1,11-Undecandiol, 1,12-Dodecandiol, Benzoesäure, Salicylsäure, Zimtsäure, Natriumbenzoat, Natriumsalicylat, Kaliumbenzoat, Kaliumsalicylat, Natriumcinnamat, Kaliumcinnamat, Dehydracetsäure, Kaliumdehydracetat, Natriumdehydracetat, Natriumsorbat, Kaliumsorbat, Sorbinsäure, 2-Phenoxyethanol, Benzylalkohol, sowie aus Mischungen von mindestens zwei der vorgenannten Verbindungen.

5. Kosmetische Zusammensetzung nach einem der Ansprüche 1 bis 4, **dadurch gekennzeichnet, dass** die Salze von C₁₂₋₂₀-Alkylphosphat, insbesondere die Salze von Cetylphosphat" in einer Gesamtmenge von 0,2 bis 2,0 Gew.-%, bevorzugt 0,3 bis 1,8 Gew.-%, besonders bevorzugt 0,3 bis 0,7 Gew.-%, jeweils bezogen auf das Gesamtgewicht der Zusammensetzung, enthalten sind.

6. Kosmetische Zusammensetzung gemäß einem der Ansprüche 1 bis 5, **dadurch gekennzeichnet, dass** die C₁₄₋₂₀-Mono- oder Diacylglyceride, bevorzugt die C₁₆₋₁₈-Mono- oder Diacylglyceride, besonders bevorzugt die gehärteten Palmölglyceride, in einer Gesamtmenge von 0,1 bis 1,7 Gew.-% bezogen auf das Gesamtgewicht der Zusammensetzung, enthalten sind.

7. Kosmetische Zusammensetzung gemäß einem der Ansprüche 1 bis 6, **dadurch gekennzeichnet, dass** die Salze von C₁₂₋₂₀-Alkylphosphat (insbesondere die Salze von Cetylphosphat) und die C₁₄₋₂₀-Mono- und C₁₄₋₂₀-Diacylglyceride bevorzugt in einem Gewichtsverhältnis C₁₂₋₂₀-Alkylphosphat-Salz zu besagtem Glycerid von 3 zu 1 bis 1 zu 1,5, besonders bevorzugt von 2,5 zu 1 bis 1 zu 1, enthalten sind.

8. Kosmetische Zusammensetzung, nach einem der Ansprüche 1 bis 7, **dadurch gekennzeichnet, dass** besagte AMPS-Polymere bevorzugt in einer Gesamtmenge von 0,005 bis 5,0 Gew.-%, insbesondere 0,005 - 2,5 Gew.-%, besonders bevorzugt 0,1 bis 2 Gew.-%, jeweils bezogen auf das Gewicht der Zusammensetzung, enthalten sind.

9. Kosmetische Zusammensetzung nach einem der Ansprüche 1 bis 8, **dadurch gekennzeichnet, dass** das besagte Polymer durch Copolymerisation von zumindest AMPS mit mindestens einem Monomeren mit schwacher Säurefunktion, das ausgewählt ist aus Acrylsäure, Methacrylsäure, Itaconsäure und Maleinsäure, erhalten wird.

10. Verwendung einer kosmetischen Zusammensetzung nach einem der Ansprüche 1 bis 9 zur Pflege der Haut, insbesondere zur Pflege von Altershaut.
